Europäisches Patentamt

European Patent Office

Office européen des brevets

(19)

(11) **EP 0 510 010 B1**

(12) **EUROPEAN PATENT SPECIFICATION**

(45) Date of publication and mention
of the grant of the patent:
**15.09.1999 Bulletin 1999/37**

(21) Application number: **91900741.9**

(22) Date of filing: **10.12.1990**

(51) Int Cl.6: **C12M 1/08**, B01J 10/00

(86) International application number:
**PCT/DK90/00324**

(87) International publication number:
**WO 91/09111 (27.06.1991 Gazette 1991/14)**

(54) **PROCESS FOR EFFECTING A CHEMICAL, BIOCHEMICAL OR BIOLOGICAL REACTION OR PRODUCTION AND A LOOP REACTOR THEREFORE**

VERFAHREN ZUR AUSFÜHRUNG EINER CHEMISCHEN, BIOCHEMISCHEN ODER BIOLOGISCHEN REAKTION ODER PRODUKTION UND EIN SCHLAUFENREAKTOR ZUM AUSFÜHREN DES GENANNTEN VERFAHRENS

PROCEDE PERMETTANT UNE REACTION OU UNE PRODUCTION CHIMIQUE, BIOCHIMIQUE OU BIOLOGIQUE, ET REACTEUR A BOUCLES DE MISE EN OEUVRE DUDIT PROCEDE

(84) Designated Contracting States:
**AT BE CH DE DK ES FR GB GR IT LI LU NL SE**

(30) Priority: **08.12.1989 DK 421689**

(43) Date of publication of application:
**28.10.1992 Bulletin 1992/44**

(73) Proprietor: **EKEROTH, Lars**
**DK-2860 Soborg (DK)**

(72) Inventor: **EKEROTH, Lars**
**DK-2860 Soborg (DK)**

(56) References cited:
**EP-A- 0 057 659**          **DE-A- 2 603 668**
**DE-A- 3 331 993**          **US-A- 4 148 691**
**US-A- 4 704 363**

• **DERWENT'S ABSTRACT, No. 90-230 455/30, SU 1 535 889, publ. week 9030.**

## Description

In a number of substance producing processes of various kinds an exchange of matter takes place between immiscible phases, e.g. solids/liquid phases, liquid/liquid phases, gas/liquid phases, gas/liquid/solids phases, or some other combinations, e.g. involving a plurality of different phases of the same kind in combination with one or more different phases of another kind. The common feature of production processes of this kind is that the exchange of matter will take place through the boundary face between the different phases, and that the amount of matter exchanged (the substance or mass transport) per unit time is, amongst others, directly related to the area of the common boundary face between the different phases involved. This can be expressed by the equation:

$$M_g \sim k \cdot a_g$$

where:

$M_g =$    the mass transport through the boundary face per unit time

$k =$    the transport coefficient, i.e. a constant independent of the boundary face area but dependent of other conditions (e.g. the concentration of the matter, the temperature, pressure, and possibly viscosity and flow conditions),

$a_g$    the area of the boundary face between the different phases involved in the exchange of matter

Usually, the production of substance per unit time will depend upon the above exchange of matter taking place between the different phases involved, whereby is meant that if this exchange of matter does not take place sufficiently rapidly or is prevented totally, that will constitute a limitation of or possibly a complete hindrance to the production of the substance or substances desired per unit time (in the following briefly designated the production rate). For this reason one will usually attempt to obtain a large boundary surface area between the phases involved, what is achieved in a number of very important industrial substance production processes by dispersion of the one phase (or optionally more phases) in the other phase(s) in the form of small bodies (particles, droplets, bubbles), and in such a case for example suspensions (solid particles in a continuous liquid phase), smokes (solid particles in a continuous gas phase), emulsions (gas bubbles or liquid droplets in a liquid phase immiscible therewith), foams (gas bubbles in a continuous liquid phase), or fog (liquid droplets in a continuous gas phase) are obtained.

The substance (or substances) being exchanged between the different phases involved can be identical to the one desired to produce in the production process in question, but it can also be a starting material or turnover substance (such as a nutrient, an energy substrate, or a respiration substance) necessary for the process or a waste material (e.g. carbon dioxide) resulting from the process, or possibly a combination of the above and/or other types of substances.

A typical field of substance production in which the above circumstances manifest themselves are the biological production processes, in which microorganisms of various kind or single cell or few cell bodies from higher animals or plants are employed for the production of various, more or less specific substances, substance mixtures, or compositions, including the microorganisms or the cells themselves. In the latter case one usually refers to the substance production as a multiplication or propagation of the culture in question, the final purpose of the culture produced being for example inoculation of another medium (e.g. souring of milk or cream), fermentations of various kinds (including e.g. the production of alcohol in vinification, beer brewing, or production of spirit), rising of dough in bread baking, or use as a fodder component or foodstuff (bioprotein), possibly after further treatment.

Viewed in a historical perspective the production of yeast, especially baker's yeast, has always been of major industrial and economical importance, and since the production process through which yeast is manufactured at industrial scale involves most of the production parametres and problems being pertinent to the present invention, it will be used predominantly in the following to exemplify and illustrate these parametres and problems as well as the principle and some embodiments of the invention.

Usually a closed container, a so-called reactor or more specifically a fermentor, is used for the production of yeast, the container being supplied a liquid (mainly water) containing or being supplied the nutrients, salts and micronutrients (together designated the substrate) necessary for the yeast, plus a starting amount of the yeast to be produced. In order that the yeast can be capable to grow and propagate it must be supplied oxygen. (If not, an anaerobic fermentation of the substrate will take place in connection with production of alcohol but resulting in only minor propagation of the yeast). In principle the oxygen can be supplied as pure oxygen gas, but since this is uneconomically expensive, atmospheric air (possibly somewhat enriched with oxygen) is in practice most often used, usually being introduced at the bottom part of the fermentor as air bubbles which of themselves, due to the general gravitation and the difference in densities between the bubbles and the surrounding liquid, will ascend through the fermentation liquid. During their ascent a diffusion of the oxygen will take place out of the air bubbles to the surrounding liquid, in which it is dissolved and from where the yeast cells will absorb it during their growth and division (propagation). The release of oxygen from the air bubbles to the surrounding liquid will gradually decrease the oxygen concentration in the bubbles, and this will reduce the transfer rate of the oxygen from the bubbles to the liquid, the more the larger the exhaustion of the oxygen in the bubbles is. This relationship will be especially pronounced in the upper part of the fermentor, in higher degrees the higher the locality is in the fermentor.

During the rise of the bubbles through the liquid they will simultaneously absorb carbon dioxide being excreted from the yeast as a waste product, and this carbon dioxide will further reduce the oxygen concentration in the bubbles and hence further reduce the oxygen transfer rate from the bubbles to the liquid, the more the higher the bubbles are in the fermentor.

Another phenomenon that limits the oxygen transfer rate from the air bubbles to the fermentation liquid is, as earlier mentioned, contingent on the size of the area of the common boundary surface between the substance exchanging phases, in this case the total surface area of the bubbles. In order to achieve a large bubble surface area one can partly make the bubbles small and partly increase the number of bubbles per unit volume of bubble-containing fermentation liquid. The mathematical product of the number of bubbles per unit volume of fermentation liquid and the average volume of the bubbles is usually expressed as the bubble volume percentage of the bubble-containing fermentation liquid and is referred to as the "hold-up value" or simply the "hold-up".

When the number of bubbles per unit volume is increased, the hold-up value is also increased and for the same size of bubbles thus also the bubble surface area and hence the oxygen transfer from the bubbles to the fermentation liquid. With increased number of bubbles per unit volume of liquid, the tendency of the bubbles towards collision with each other and subsequent fusion (bubble coalescence) is, however, also increased, which leads to larger bubbles, smaller bubble surface area, and hence reduced oxygen transfer rate.

In most fermentation liquids the tendency towards bubble coalescence becomes almost instantaneous at hold-up values above approximately 30%, which therefore in practice usually represents the upper limit for increasing the oxygen transfer rate through increase of the hold-up in the fermentation liquid. The degree of bubble coalescence occured will moreover be the larger the higher one moves upwards in the fermentor, as a consequence of the fact that the probability of a bubble having collided with another bubble increases with the length of the path traveled by the bubble, which length will be related to the height of ascension.

Both the increasing extent of bubble coalescence and the earlier mentioned decrease of the oxygen concentration in the air bubbles with the height in the fermentation liquid will thus cooperate on a reduction of the oxygen transfer from the bubbles to the liquid, the more the higher one moves up in the fermentor. Actually, for ideally small bubbles one does not have to go up very far in the fermentor, before the oxygen transfer is strongly reduced and approaches zero. From this level and up the propagation of the yeast will be extremely limited, and the superjacent fermentation volume will in reality be a dead space as viewed from a point of yeast production. For this reason, the height of a fermentor would be severely limited, if at the bubble formation stage ideally small bubbles were produced, which would give the largest obtainable oxygen transfer and thus the highest possible production rate, since the oxygen transfer rate is in fact the limiting factor for the propagation rate. In industrial production, where it is usually desired to use fermentor sizes from about 10 to 1,000 $m^3$, one is forced, however, for practical reasons to use fermentors of a considerable height and is therefore forced to generate larger bubbles at the formation and thereby a more severe limitation of the oxygen transfer rate at the bottom of the fermentor in return for having some oxygen supply available further up in the fermentor.

Notwithstanding this non-ideal compromise solution to the problem of providing sufficient oxygen, both at the bottom of the fermentor and further up in the fermentor, some portion of the culture will obtain less oxygen (without any portion obtaining an optimum oxygen supply). Hitherto this problem has been attempted remedied by two fundamentally different types of fermentor constructions, viz. the stirred fermentor type and the loop fermentor type.

In the stirred or back-mix fermentor or reactor type one or more stirring means will be provided in the fermentor. Possibly they may be positioned at different heights in the fermentor, but at least one will usually be located at the fermentor bottom. Through stirring with the stirring means the fermentation liquid will be homogenized in all directions and levels at such powerful stirring that strong turbulence and vertical circulation in the fermentation liquid is caused. Hereby a new problem is created, however, in as much as the circulation and turbulence of the liquid in the up- and downwards direction as viewed from a statistical point will enhance the ascension of some bubbles through the liquid to such a degree that they reach the liquid surface uppermost in the reactor, before they have had time enough to release so much of their original content of oxygen as would otherwise for energy consumption and economical reasons be appropriate to exploit, whilst the ascension of other bubbles will be impeded to such a degree that they are maintained in the liquid long after they have released that part of their original oxygen content, which it for energy consumption and economical reasons is reasonable to exploit, for which reason they constitute a reactor volume-occupying dead space. Additionally, the powerful stirring will enhance the tendency towards bubble coalescence amongst bubbles of equal oxygen concentration as well as bubbles of unequal oxygen concentration. Aeration of fermentors of the stirred type is thus by no means ideal and far from optimum.

The other type, the loop fermentor or reactor type, is characterized by the fact that the fermentation liquid has a predominant mass flow (bulk flow), which essentially has the character of piston or plug flow, through at least one closed or endless cycle (the loop) which is defined by solid physical bounderies such as container, tube and/or hosepipe walls which are connected into an annular system.

That the mass flow has the character of piston or plug flow does not mean that the flow necessarily has to be laminar, it can very well be turbulent and even with some inner circulation or back-mix, as long as the underlying tendency towards inner backflow in the cycle does not reach the same magnitude as the predominant mass flow in the flow direction of the cycle.

That the mass flow takes place in a closed or endless cycle means that the prevailing or at least an essential part of the fermentation liquid at least at one position of the cycle passes through the cross section of the cycle at least twice at this position, before it leaves or is withdrawn from the cycle. En route fermentor liquid may well be introduced into or removed from the cycle to some minor extent at one or more positions, and this is so far as it goes the usual practice. The introduction and removal flows procured hereby should just not become dominant compared to the main mass flow in the cycle.

When the volume elements of the fermentation liquid wnich at a given time are present in a given cross sectional layer of the cycle (the loop) theoretically for the first time again, due to the average velocity of the predominant mass flow, are located in this cross sectional layer, one passage of the cycle is said to have taken place.

The mass flow can take place in an essentially horisontal or vertical direction or any direction therebetween, the essentially vertical direction probably being the most common.

In its simplest embodiment a vertically oriented loop fermentor can be composed of a cylindrical container closed at both ends, in the interior of which a shorter cylindrical tube having a smaller diameter than the container and having both ends open is located coaxially with the cylinder axis of the container. The circulation of the fermentation liquid occurs as a mass flow in one direction within the cylindrical tube and a mass flow in the opposote direction in the annular space between the cylindrical tube and the cylindrical wall of the container, these mass flows joining each others at the ends of the inner cylindrical tube which, incidentally, usually is referred to as a draught or draft tube. In principle, the mass flow can be generated by a mechanical impeller, such as a turbine, a screw, or a propeller, being positioned suitably in the cycle. In practice, however, the mass flow will usually be generated by means of the aeration air and possibly supplementary liquid being supplied to the fermentor, which air and optional liquid is then introduced into the fermentor at great flow speeds as momentum flows, optionally a combined one, at a suitable position in the fermentor. Loop fermentors (especially those with an inner draft tube in a cylindrical container) of various designs have in great numbers been described in the patent literature, being operated both mechanically, pneumatically or hydraulically or through combinations thereof; reference is made just to for example US 3,910,826, US 3,984,286, US 4,036,699, and DE 2,603,668.

In a vertically oriented loop fermentor having e.g. one cycle the aeration bubbles can in principle be introduced anywhere in the cycle, but as a consequence of the fact that they usually always have to be withdrawn at the top of the cycle due to the natural tendency of the bubbles to accumulate, coalesce, and separate from the liquid at this place, the introduction of the bubbles will usually be at the bottom of the cycle. Thus, this is the case for most draft tube loop fermentors, wherein the air, possibly together with supplementary liquid, is supplied as a momentum flow into the lower end of the draft tube. The introduction of air bubbles into the loop fermentor is not necessarily unproblematic, but in the course of time many technical solutions of how to produce both bubbles of a desired, optionally relatively uniform, size and having the momentum wanted, i.e. the product of mass and flow speed. In particular, both very small bubbles (bubble diameters even below 100 µm) and an inlet flow of great momentum can be achieved by a "liquid-jet-nozzle gas ejector", to which air and supplementary liquid under pressure are both being supplied.

Until now the greatest problem in aerated loop fermentors has been, however, the removal of introduced air bubbles, because, of course, after a certain residence time in the fermentation liquid these bubbles will have released their oxygen content or at least that part of it which it is appropriate to exploit. As mentioned above, the natural removal of air bubbles will be at the top of the fermentor cycle. This does usually imply, however, that here there is a liquid-free space (head space), cf. for example the earlier mentioned US 3,910,826 and 3,984,286, which, however, will become partly or totally filled up with foam, for which reason the actual deaeration in reality becomes an exhaust of foam. The foam liberated must of course be broken somehow, since otherwise completely unacceptable environmental problems in the surroundings would arise, as would unacceptable production losses occur. Traditionally, foam problems have been attempted to be controlled by the addition of foam suppressors or by reduction of the concentration of ions and surface active substances. Addition of foam suppressors will, however, increase the bubble coalescence drastically down in the fermentation liquid. A reduction of the ion concentration and/or the concentration of surface active substances may have a negative influence on the fermentation, especially the propagation rate.

According to DE 3,331,993 it has been tried, however, to avoid the foam formation problem by letting the air be liberated from the air/liquid phase system (the fermentation liquid) only in such a powerful centrifugal field that no foam can be formed. In practice this is effected by letting the fermentor be completely filled with liquid and contiuously withdrawing a minor branch stream from the upper end of the reactor. This branch stream is sent to an outer cyclone separator, in which liquid and air are effectively separated and liberated air vented to the atmosphere, while deaerated liquid is possibly being recirculated to the reactor.

According to EP 51,151 it is not attempted to avoid the formation of foam. It is merely teached, how formed foam can be broken at the same time as the risk of contamination of the fermentation liquid can be minimized. This is realized by mounting a centrifuge in the interior of a fermentor, the centrifuge being supplied the foam that accumulates in the upper part of the fermentor through a funnel mounted on the top of the centrifuge. In the centrifuge the foam is separated into a cell-rich liquid phase and a liquid phase containing gas, or possibly into a cell-rich liquid phase, a cell depleted liquid phase, and a gas phase, of which at least a part of the liquid phase and/or the cell-rich phase is withdrawn from the fermentor. The fermentor used according to the EP patent is, however, not a loop fermentor but rather a stirred fermentor with complete back-mix. The retention time is specified as 8 to 10 hours.

Thus, neither DE 3,331,993 (nor EP 51,151) does give any directions of how to solve the earlier mentioned, serious problem existing in loop fermentors, namely how to get rid of the introduced but progressively oxygen depleted air bubbles. Those air bubbles which will first and foremost be separated from the liquid in the uppermost part of the cycle of a loop fermentor will be the largest bubbles, since these will have the largest buoyancy and hence the highest speed of rising, which will give them the greatest capability for overcoming and counteracting the descending liquid movement following the ascending liquid movement in the circulation flow. Minor and small bubbles will, to a higher degree, be carried along in the circulation flow, one cycle passage after the other, and only due to enlargement through bubble coalescence or inward diffusion of carbon dioxide they will gradually reach the bubble size leading to separation uppermost in the cycle. Minor and small bubbles are, however, as mentioned earlier, the most effective oxygen transporters, in as much as they more rapidly release their oxygen to the surrounding liquid. In the loop fermentors hitherto known they are, however, not ideal, since they will be dragged around in the cycle as useless, space-occupying dead volumes after their rapid release of the oxygen.

The present invention provides a teaching as to how minor and small air bubbles ideal as oxygen transporters can be used in loop fermentors and at the same time having them removed from the circulation at the time, where their oxygen content has been reduced to a level that judged from an oxygen transportation point of view makes them no longer optimal or appropriate. The invention thus provides the possibility of fulfilling the oxygen demand of a loop fermentor to a hitherto unattainable degree and thereby obtain a hitherto unattainable productivity per unit volume of fermentation liquid. The invention provides, however, also productivity advantages when larger air bubbles are used, as the removal of oxygen depleted or oxygen-poor bubbles will be more effective also for such larger bubbles, whereby a greater part of the circulation volume will be available for production, which will increase the fermentor's productivity per unit volume of fermentation liquid.

This is achieved by the process of the invention for effecting a chemical, biochemical, or biological reaction or production in a loop reactor, in or into which immiscible phases are produced or introduced, respectively, during the circulation of the contents of the reactor, which process is characterized by the fact that for each cycle passage through the loop all or essentially all the reactor contents are passed through a centrifugal seperator for generation of at least two fractions having different contents of at least two immiscible phases, one of which fractions continues through the loop, while the other is drained off the loop.

The present invention also concerns a loop reactor for performing the said process, which loop reactor comprises at least one loop with a reaction volume, propulsion means for operating the circulation in the loop, and a centrifugal separator, which reactor being characterized in that the centrifugal separator is an integral part of the loop, thus receiving all or essentially all the reactor contents for each cycle passage through the loop, whereby the contents are divided into at least two fractions having different contents of at least two different constituents, and that the centrifugal separator has means for separation of the two fractions, means for passing one of the fractions further on in the loop, and means for draining off the other of said fractions from the loop.

The invention will be further described in more detail with reference to the drawings, wherein:

Figure 1 is a longitudinal sectional view executed through the axis of an embodiment of an inner loop fermentor or reactor according to the invention,

Figure 2 is a cross sectional view of two combined spiral and helical channels in a cyclone separator according to the invention, taken along the line A-A in Figure 1,

Figure 3 shows a cross section in an enlarged scale of a combined nozzle device for air and liquid introduction into a loop fermentor or reactor according to the invention, taken along the line B-B in Figure 1,

Figure 4 shows, in approximately the same scale as in Figure 3, a cross section taken along the line C-C through the nozzle device shown in Figure 3,

Figure 5 represents a longitudinal sectional view executed through the axis of a second embodiment of an inner loop fermentor or reactor according to the invention,

Figure 6 represents a cross sectional view of an inner loop fermentor according to the invention, taken along the line D-D through the embodiment shown in Figure 5,

Figure 7 represents a longitudinal sectional view executed through the axis of a third embodiment of an inner loop fermentor or reactor according to the invention,

Figure 8 represents a cross sectional view taken along the line F-F through the embodiment of an inner loop fermentor or reactor shown in Figure 7, which cross section shows a helical channel in the cyclone separator as seen from above,

Figure 9 shows a guiding plate in the helical channel shown in Figure 8, as seen from the side,

Figure 10 represents a longitudinal sectional view executed through the axis of a fourth embodiment of an inner loop fermentor or reactor according to the invention,

Figure 11 represents a cross sectional view taken along the line G-G through the embodiment of the inner loop fermentor shown in Figure 10,

Figure 12 shows in an enlarged scale a helical channel as seen from above of a cross section taken along the line H-H in the loop fermentor shown in Figure 10,

Figure 13 shows, in a flat version before being bent to the screw-shape, one of the guiding plates in the helical channel shown in Figure 12,

Figure 14 shows the screw channel guiding plate shown in Figure 13 as seen from the side after being bent to its screw-shape,

Figure 15 represents a longitudinal section executed through the axis of a fifth embodiment of an inner loop fermentor or reactor according to the invention,

Figure 16 represents a longitudinal section executed through the axis of another embodiment of the inner loop fermentor or reactor shown in figure 15, which embodiment is closed at the bottom and has one or more exits at the top for liberated air or air-mixed liquid,

Figure 17 represents a longitudinal section executed through the axes of an outer loop fermentor or reactor according to the invention.

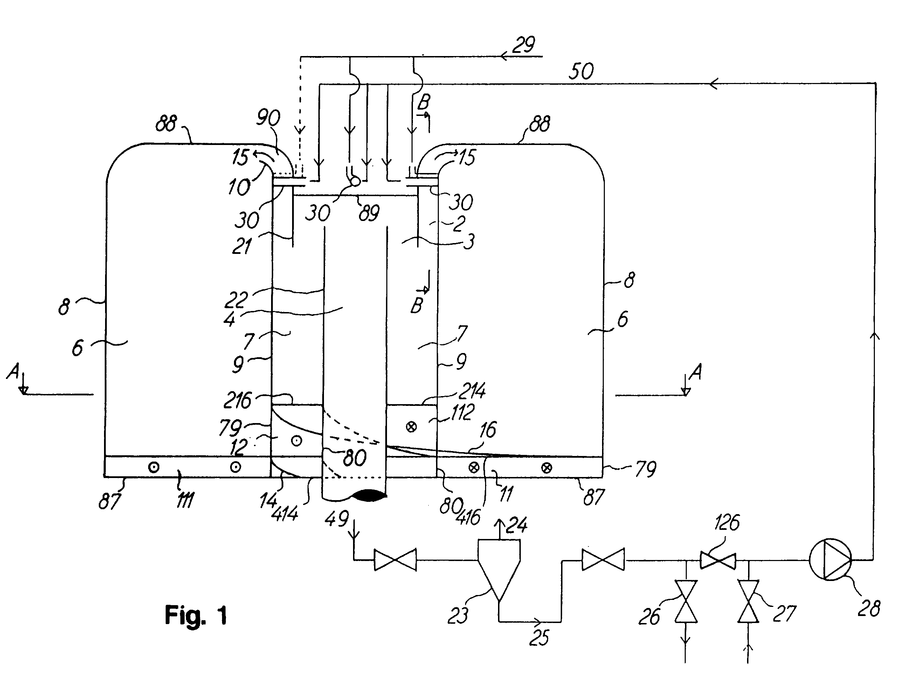

The embodiment of an inner loop fermentor or reactor according to the invention depicted in Figure 1 in an axially longitudinal sectional view has an outer container wall 8 and an inner container wall 9 which together with the adjacent bottom wall 87 and top wall 88 define an outer, cylindrical-annular reaction or working volume 6 of a fermentor loop. An inner cylindrical tube 22 defines, together with the inner container wall 9, the bottom wall 87, and a head cover 89, an inner cylindrical-annular separation volume of the fermentor loop. The outer working volume and the inner separation volume are connected at the top via an annular gateway 90, as the inner container wall 9 does not at this place extend all the way up to the top wall 88 of the container. At the bottom, the working and the separation volumes are connected via 180° gateways through the inner container wall 9, each of these passages (gateways) having a channel-shaped elongation extending into the outer working volume. One of these channel extentions is depicted in the Figure and is established by a vertical curved guiding plate 79, the container bottom wall 87, a lower guiding plate 14, and an upper guiding plate 16. Each channel extension is elongated into the inner separation volume, where the one channel extension shown in the figure is defined by the upper guiding plate 16, the lower guiding plate 14, and a vertical curved guiding plate 80. The upper guiding plate 16 has a lower introductory edge 416 and an upper terminating edge 216 and the lower guiding plate has a lower introductory edge 414 and an upper terminating edge 214.

The upper guiding plate 16 and the lower guiding plate 14 are, up to the upper terminating edge 216 of the upper guiding plate, snail-shaped and are turned upwards, so that the channel defined hereby takes the shape of a snail passage, i.e. a spirally and helically shaped channel, the debouchment of which in the inner separation volume is designated 12. A similar snail passage (not shown) is located in the space in front of the plane of the paper in the Figure and has the debouchment 112 shown in the Figure.

During operation, air, possibly pressurized, and liquid are supplied via conduit 29 and conduit 50, respectively, through the nozzle devices 30. The nozzles deliver the liquid at great speed in an inclined upward-outward direction 15 in the annular passage between the outer reaction volume 6 and the inner separation volume 7, and hereby it tears out the air supplied into small bubbles. The combined air and liquid flow acts as a momentum flow which is deflected in downward direction by the top wall 88 and the outer wall 8 of the fermentor. As a consequence of the inclined positioning of the nozzles the momentum flow will, however, not only have a radial velocity but also a tangential velocity which gives rise to a turbulence field created of gyroscopic forces in the annular gateway 90 at the bending 10 of the free end of the inner container wall 9, which turbulence field will reduce the radial velocity of the flow to some extent. The liquid flow in the working volume 6 may, dependent on the speed and inclination of the momentum flow, become turbulent, turbulent with rotation about the inner container wall 9, or turbulent with rotation and inner circulation, but regardless of these conditions the overall flow will be downwards and have the character of piston or plug flow. At the bottom of the outer working volume 6 the fermentation liquid is led into the openings 11 and 111, respectively, of the snail-shaped channels which lead to the inner separation volume 7. In case the fermentation liquid has a rotation about the inner container wall 9, this rotation will have such direction that it leads the liquid directly into the openings 11 and 111, respectively. The snail-shaped (i. e. spiral and helical) channels will amplify this rotation, and the rotational velocity of the liquid will be increased strongly as a consequence of the gradually decreasing radius of curvature. Even if the fermentation liquid flowing downwards in the outer working volume does not have any rotation about the inner container wall 9, it will nevertheless be given a rotation during the passage through the openings 11 and 111, respectively, and, as a consequence of the gradually decreasing radius of curvature, an increased rotational velocity. Since the snail-shaped channels have considerably smaller cross-sectional passages than the cross-sectional passage of the outer working volume, the pressure height prevailing at the bottom of the working volume will be transformed into a speed which will accelerate the speed of the liquid through the snail-shaped channels, which will also contribute to acceleration of the rotational velocity of the liquid in the inner separation volume round the inner cylindrical tube 22. The combined effect of these flow parametres will cause the liquid to be given a strong upwards directed helical rotation about the inner cylindrical tube 22, when it leaves the snail-shaped channels through their exit openings 12 and 112, respectively, in the inner separation volume 7. This rotation about the inner, cylindrical tube 22 will create a centrifugal field in the inner separation chamber 7, and this centrifugal field will cause the heavier components of the fermentation liquid to seek outwards towards the outer wall 9 of the inner separation chamber 7 and the lighter components, such as air bubbles, of the fermentation liquid to seek inwards towards the inner wall, i.e. the inner cylindrical tube 22, of the separation volume 7. The combined result thereof will be that at the lower edge of a cylindrical separatory wall 21, which is fastened to the head cover 89 of the inner separation volume 7, the fermentation liquid will be divided into an outer cylindrical ring-layer consisting of essentially bubble free liquid and cells and an inner layer consisting of air and/or strongly air bubble-containing liquid with essentially unchanged density of cells in the liquid. As a consequence of the downwards protruding, cylindrical separatory wall a physical separation of the two layers will now take place, as the bubble free liquid layer will be passed up through the cylindrical ring-passage 2 between the inner container wall 9 and the cylindrical separatory wall 21, while the inner air layer and/or strongly air bubble-containing liquid layer will be passed up through the annular opening 3 between the cylindrical tube 22 and the downwards protruding cylindrical separatory wall 21 and by the cover wall 89 forced downwards through the interior 4 of the cylindrical tube 22. An effective removal of the air bubbles delivered by the nozzle devices 30 has thus been achieved already after the first passage of these through the cycle of the loop fermentor, which is rendering the use of, as oxygen transporters, ideally small air bubbles possible, and (more or less) oxygen depleted air bubbles will essentially not be carried on in the cycle of the loop fermentor. The cylindrical tube 22 serves, in addition to being an outlet means from the fermentor, as a central space-occupying means in the separation volume 7, so that no region subjected to a low G.-value and hence a poor separation exists in center of the separation volume.

The fermentation liquid in the cylindrical ring-passage 2 will, under continued rotational upwards directed movement, be passed up into the upper annular gateway 90, where the liquid will be imparted new increased flow speed or higher pressure by the momentum flow of liquid and torn-out bubbles produced by the nozzle devices 30 and, as described above, be passed into the outer working volume 6 of the fermentor and thereafter continue its flow as described previously.

The flow of air and/or strongly air bubble-containing liquid passed into the interior of the cylindrical tube 22 will be led downwards through the tube and via a conduit 49 passed to an outer separator 23 which may be a gravitational separator or a cyclone separator or a centrifuge, in which air and fermentor liquid will be effectively separated. Liberated air 24 will be vented into the atmosphere, possibly after a further purification, while extracted liquid will be led via conduit 25 and possibly drained off via valve 26 or passed on via the valve 126 to the pump 28 which, at a sufficient pump pressure, will return it via conduit 50 to the nozzle devices 30. Supplementary liquid containing nutrients, salts, and micronutrients necessary for the culture may via the valve 27, the pump 28, and conduit 50 be passed to the nozzle devices 30 for maintenance of the amount of fermentor liquid necessary for propagation of the culture.

As is evident, the embodiment of the loop fermentor or reactor according to the present invention shown in Figure 1 is an inner loop fermentor, i.e. a loop fermentor wherein the one part of the cycle is completely surrounded by the other part of the cycle. As can further be seen, the inner part of the cycle constitutes an effective cyclone separator while the outer part of the cycle constitutes a reaction volume, in which the uptake and the turnover of oxygen is essentially taking place. Naturally, however, there will also be some turnover of the oxygen in the separation volume, i.e. the cyclone separator, during the separation of the more or less oxygen depleted air bubbles.

In the embodiment shown, the two channel elongations will maintain the radial orientation of the liquid, which is partly rendering the level of turbulence low for the benefit of the separation and partly enabling some pre-separation in the reaction volume 6. This renders the use of smaller bubbles and hence higher productivity possible.

In accordance with the Figure, fermentor liquid is exhausted via conduit 49 and possibly drawn off via the valve 26. In a continuously operating fermentor this off-drawing could constitute the flow of product, but in addition a direct outlet for drawing off product, both at continous and batch operations, may be provided in the fermentor.

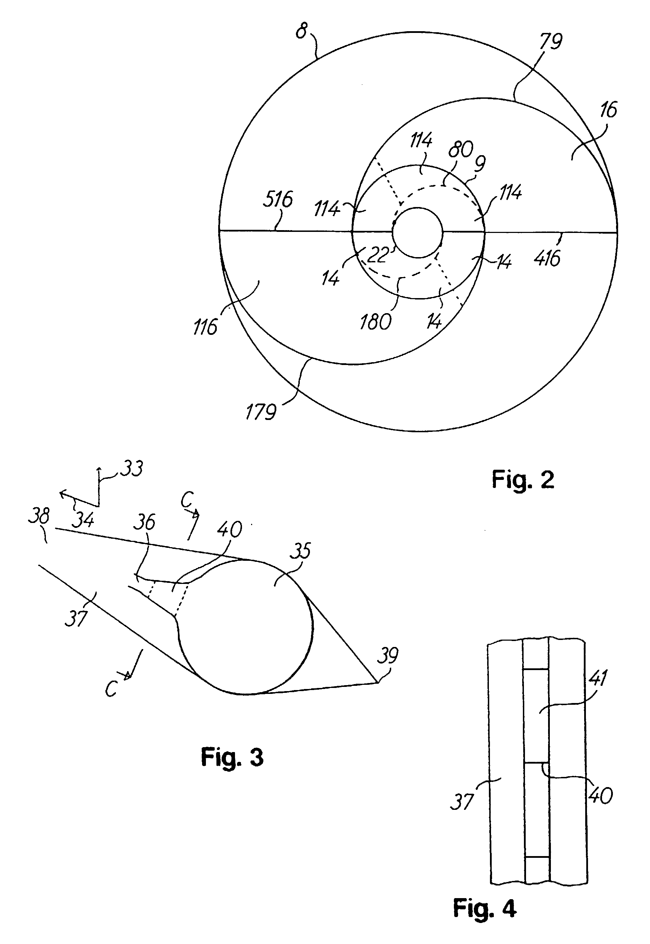

Figure 2 shows a cross sectional view taken along the line A-A in the loop fermentor according to the present invention shown in Figure 1. In the Figure, the bottom part of the fermentor is viewed from above. 8 Indicates the outer container wall of the fermentor, 9 indicates the inner container wall, 22 indicates the inner cylindrical tube, 14 and 114, respectively, indicate the lower guiding plates, 16 and 116, respectively, indicate the upper guiding plates, 79 and 179, respectively, designate the outer guiding plates, and 80 and 180, respectively, designate the inner guiding plates of the two spiral- and helical-shaped channels leading from the outer working volume to the inner separation volume of the fermentor, and 416 and 516, respectively, indicate the upper, terminating edges of the upper plates 16 and 116, respectively.

Figure 3 shows a section through a nozzle device for combined introduction of air and liquid, the latter or both pressurized, said section being executed along the line B-B through the nozzle device 30 shown in Figure 1. The nozzle device has an inner, tubular chamber 35 for introduction of liquid and an outer chamber 37 for introduction of air. 40 Indicates a cross wall in the slit-shaped outlet nozzle 36 of the liquid chamber for keeping together the walls of the outlet nozzle when the pressure is high. 39 Indicates a crest-shaped edge for limitation of the turbulence in the liquid surrounding the nozzle device 30. During operation, liquid is expelled from the liquid chamber 35 out through the nozzle 36 inside the air chamber 37 and further out through the slit-shaped debouchment 38 of the device out into the fermentation liquid in the reaction volume of the fermentor. Hereby the air in the chamber 37 is torn out into a flow of small air bubbles which together with the liquid expelled by a high pressure attain a high velocity and hence constitute a momentum flow that acts as a propulsion force for the circulation in the fermentor. In the arrow angle shown above the nozzle, 33 indicates the vertical direction and 34 the direction of the flow of fermentor liquid at the position of the nozzle device in the fermentor.

Both the positioning of the nozzle devices in the annular passage 2 and their inclination reduce the number of nozzle devices necessary for operating the fermentor.

That the nozzles are slit-shaped and essentially span all across the passage 2 saves energy in the bubble formation and gives a very uniform spreading of the bubbles in the liquid.

Figure 4 shows a cross section taken along the line C-C through the nozzle device shown in Figure 3. 41 Indicates the outlet slit of the liquid chamber, 37 indicates the air chamber, and 40 indicates cross walls for keeping together the outlet slit of the liquid chamber.

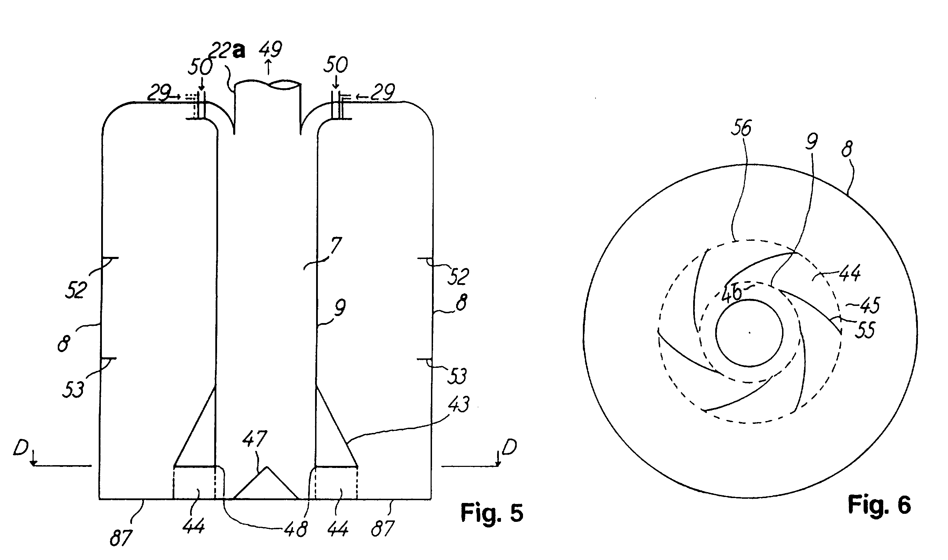

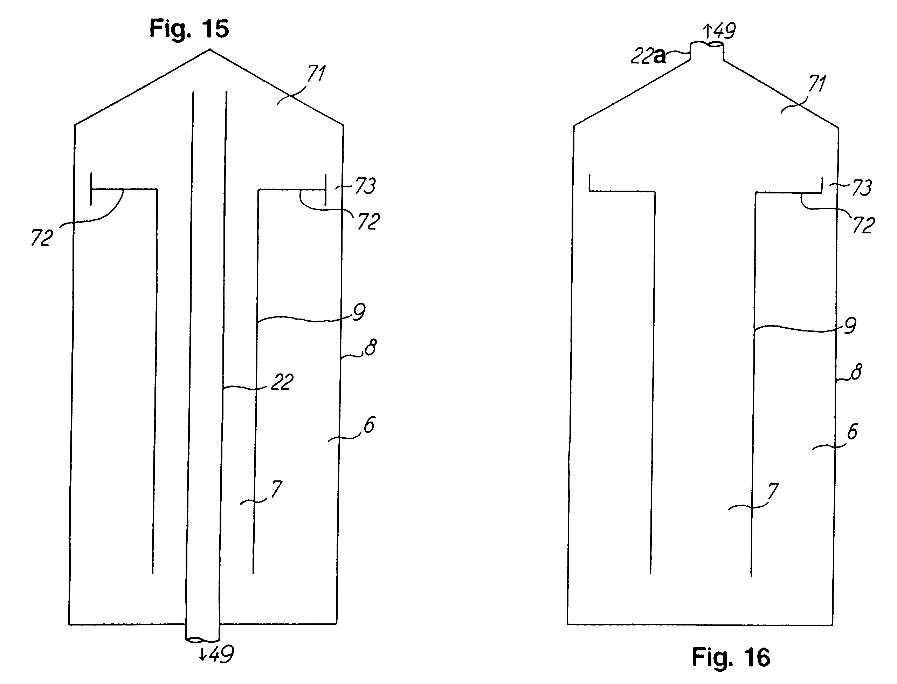

Figure 5 represents a longitudinal sectional view executed through the axis of a second embodiment of an inner loop fermentor according to the present invention. This fermentor has spiral-shaped channels 44 that are not helical and the outlet 22a from the inner centrifugal separator of the fermentor is placed at the upper end of the separator, so that outlet of air and/or air bubble-rich liquid separated in the centrifugal separator takes place in an upward direction. The outlet conduit can in its initial part be made conical as the fermentor top in Figure 16, and a distinct, preferably annular, return conduit for liquid to the reaction volume 6 may be employed.

The spiral channels 44 at the bottom of the fermentor are defined by askew positioned plates, the bottom wall 87 of the fermentor, and a ring-cone at the outside of the foot of the inner container wall 9. A conical elevation placed centrally on the bottom plate 87 inside the separation volume 7 forces the fermentation liquid passed in through the spiral channels, upwards around the inner circular upper edge 48 of the spiral channels 44, so that the liquid is given an upwards directed rotation in the separation chamber 7. Hereby a cyclone separation effect similar to the one referred to under Figure 1 is achieved.

The reference numbers 52 and 53 indicate two rims of flow regulating guiding plates placed on the outer container wall 8, which guiding plates are directed obliquely upwards to the right and upwards to the left, respectively, and, in the case of tangential rotation in the reaction volume 6, have the purpose of contributing to the creation and/or maintenance of relatively weak radial whirls in the downgoing liquid flow in the outer reaction volume, what will counteract separating out of bubbles in the reaction volume. Plates could be mounted obliquely in other ways in the reaction volume 6 and produce the same result. The plates 52 and 53 shown are examples of good positioning. Other reference numbers in Figure 5 have the same meaning as mentioned in Figure 1.

Figure 6 shows a cross sectional view taken along the line D-D through the loop fermentor shown in Figure 5. The Figure thus shows the bottom part of the loop fermentor shown in Figure 5 viewed from above. 8 Indicates the cylindrical outer wall of the loop fermentor and 9 indicates the cylindrical inner wall of the fermentor. 56 Designates the lower outer edge of the ring-cone placed around the inner cylindrical wall, 44 designates a spiral channel, 45 the entrance of this channel, and 46 its debouchment into the inner separation volume, whilst 55 indicates a side wall in the spiral channel.

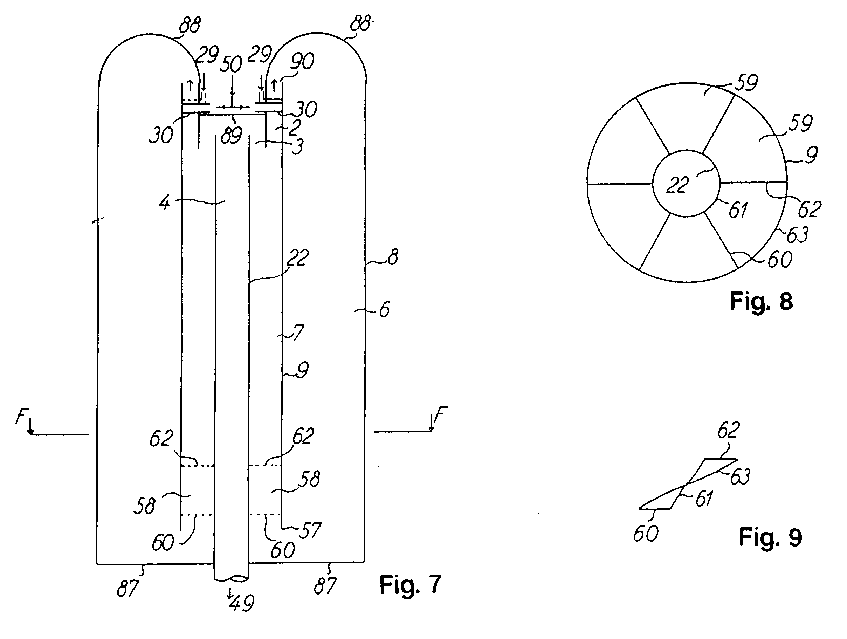

Figure 7 shows a longitudinal sectional view executed through the axis of a third embodiment of an inner loop fermentor according to the present invention. This fermentor has connection channels 58 between the outer reaction volume 6 and the inner separation volume 7, which channels 58 are helical but not spiral-shaped. In the Figure, 60 indicates the introductory edge of a screw channel guiding plate and 62 the terminating edge of a screw channel guiding plate. 57 Indicates the lower free edge of the inner cylindrical container wall 9. Other references have the same meaning as in Figure 1. The fermentor circuit can be provided with one or more rims of obliquely mounted guiding plates as mentioned in connection with Figure 5.

That the connection channels 58 are surrounded by the inner container wall 9 means that these channels, in contrast to the spiral channels in Figure 5, are placed over the loop bottom, where the liquid attains its upward velocity. Hereby the connection channels 58 can dampen the turbulences created at the loop bottom, thereby improving the subsequent separation.

The tall design of this third embodiment is advantageous for processes, where pronounced tendency to coalescence of bubbles or some other reason dictates the use of relatively large bubbles. Large bubbles ascend faster, and since the bubbles are to be brought downwards by the liquid in the outer reaction volume 6, the height is advantageous by increasing the downflow speed of the liquid in the reaction volume.

Figure 8 shows a cross sectional view taken along the line F-F through the loop fermentor shown in figure 7, but only the inner part delimited by the inner, cylindrical wall 9 of the fermentor. 22 Indicates the inner cylindrical tube, 59 indicates a screw channel guiding plate or ascent plate, 60 indicates the introductory edge of a screw channel guiding plate, 61 indicates the inner edge of a screw channel guiding plate, 62 indicates the terminating edge of a screw channel guiding plate, and 63 indicates the outer edge of a screw channel guiding plate.

Figure 9 shows one of the screw channel guiding plates of Figure 8 shown from the side. 60, 61, 62, And 63 indicate the same as in Figure 8. The plate is twisted out of plane but is not curved, since this is not necessary because the embodiment of the loop fermentor according to the present invention shown in Figure 7 does not contain means that will brake the tangential rotation in the outer cycle, which means that the fermentation liquid will be in rotational motion before being introduced into the screw-shaped channels leading to the inner separation chamber. The plate is twisted out of plane in order to secure essentially uniform angular velocity in the radial dimension of the separation volume 7. This minimizes the level of turbulence in the separation volume, which improves the separation of bubbles from the liquid.

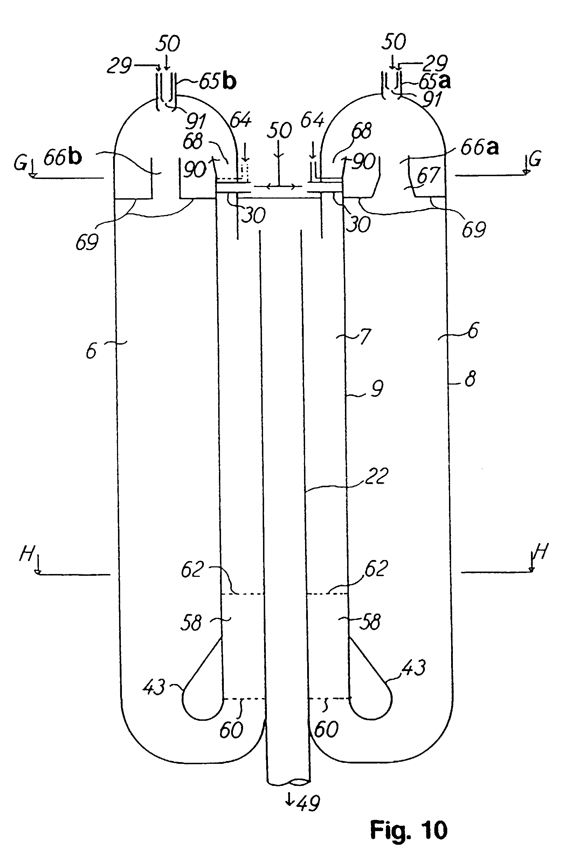

In this relatively slender design of the loop fermentor a number of rims of oblique guiding plates, as in Figure 5, will, however, typically be advantageous, and if mounted they will give a somewhat stronger braking of the tangential rotation in the outer reaction volume 6. If the tangential rotation is braked in this way or by other means, e.g. vertical-radial guiding plates in the outer reaction volume 6, then it can be advantageous to curve the screw channel guiding plates to a larger or lesser degree, for example by approaching their shape to the shape of the screw channel guiding plates in the embodiment of the invention shown in Figure 10.

Figure 10 shows a longitudinal sectional view executed through the axis of a fourth embodiment of an inner loop fermentor according to the present invention. This fermentor has, in addition to nozzle devices 30 for the introduction of gaseous material 64 and liquid 50, an extra set of inlet devices 65a and 65b for introduction of air 29 and liquid 50. Furthermore, the fermentor has a rim of momentum exchange tubes 66a and 66b positioned in line with the debouchments of the extra liquid inlet devices, while the upper part of the reaction volume 6 is otherwise essentially blocked by a plate 69, so that all or nearly all downward flow will have to pass through the momentum exchange tubes 66a and 66b. The liquid jet from inlet device 65a will tear away any gas accumulation at the top of the fermentor. The liquid nozzle 91 in the inlet devices 65 can be essentially circular, oblong or star-shaped, just as there can be more than one nozzle in each inlet device.

In the embodiment shown the momentum exchange tubes are parallel to the axis of the fermentor, whereby the rotation of the fermentation liquid around the axis after its passage through the inner centrifugal separator is braked by the momentum exchange tubes in the upper part of the reaction volume. As a consequence of this the initial part of the helical channels 58 between the reaction volume 6 and the interior of the separation volume 7 is made parallel to the axis of the fermentor.

The momentum exchange tubes 66a and 66b do not, however, have to be parallel to the axis of the fermentor, and the extra inlet devices for liquid and air can be used without the momentum exchange tubes 66a and 66b and/or the blocking plate 69 positioned uppermost in the reaction volume 6.

Braking of the rotation of the fermentation liquid in the downstream part of the cycle (the reaction volume 6) can also be accomplished with plates parallel to the axis of the fermentor.

The momentum exchange tube 66a is provided with a diffuser 67, which increases the momentum transfer to the circulation through the cycle.

The momentum exchange tube 66b (which is not provided with a diffuser) and the blocking plate 69 have the same purpose.

Also the annular passage 90 is shown with a diffuser 68 that causes a pressure rise in the liquid flowing through the passage 90, which contributes advantageously to the flow of the liquid from the inner separation chamber 7 to the outer reaction volume 6.

In the nozzle devices 30, liquid 50 from the pump 28 (not shown) or a pump parallel therewith can be introduced, as can e.g. $NH_3$, $O_2$ and/or $CH_4$-containing gas, e.g. natural gas or biogas, through conduit 64.

That the inlet means 29 for air are placed after the nozzle devices 30 in the loop means that the gas from conduit 64 can be comminuted into finer bubbles than the air comminuted into bubbles at the inlet devices 65a and 65b without increasing the risk of coalescence of air bubbles with bubbles of gas from conduit 64. This allows for higher exploitation of the typically more expensive gasses from conduit 64.

At the lower part of the fermentor the inner container wall 9 is shown provided with an outwards bent free end 43, cross-sectionally shaped like a pear, while 58, 60, and 62 as previously indicate screw-shaped channels from the outer to the inner cycle of the loop fermentor, the introductory edge of a screw channel guiding plate (ascent plate), and the terminating edge of a screw channel guiding plate (ascent plate), respectively.

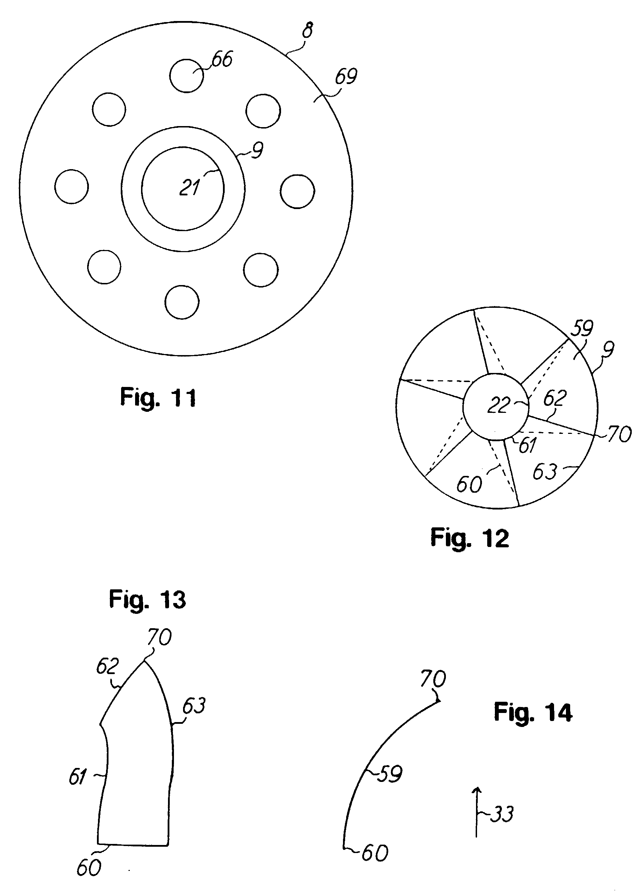

Figure 11 shows a cross sectional view taken along the line G-G through the loop fermentor shown in Figure 10 and thus shows the blocking plate 69 and the momentum exchange tubes 66 (66a and 66b) seen from above. The reference numbers 8, 9, and 21 have the same meaning as previously stated.

Figure 12 shows a cross sectional view taken along the line H-H through the loop fermentor shown in Figure 10, but only the inner part laying inside the limitation defined by the inner container wall 9. 22 Indicates the inner cylindrical tube, 59 indicates the guiding plate (the ascent plate) in a screw-shaped channel between the outer reaction volume and the inner separation volume, 60 indicates the introductory edge of a guiding plate (ascent plate), 61 indicates the inner edge, 62 indicates the terminating edge, and 63 indicates the outer edge of such a plate. 70 Indicates a corner between a terminating edge and an outer edge of a guiding plate.

Figure 13 shows a screw channel guiding plate according to Figure 12, cut out of a flat plate and before being curved for use in the screw-shaped channel. The reference numbers 60, 61, 62, 63, and 70 have the same meaning as stated under Figure 12.

Bending of the plate before insertion into the channel can adequately be performed over a cylinder. Circular curvature of the plate gives a steady acceleration in the tangential direction, and when the acceleration is steady the level of turbulence is minimized for the benefit of the subsequent separation process.

That the introductory edges 60 of the screw channel guiding plates are positioned at an angle to radial direction provides a more uniform angular velocity, cf. the text to Figure 9.

Figure 14 shows the screw channel guiding plate from Figure 13, seen from the side after being bent for use in the screw-shaped channel in the loop fermentor in Figure 10. The plate is bent in a plane-circular manner, which is sufficient to achieve good functionality. The reference numbers 60, 59, and 70 have the same meaning as in figure 12, and the arrow 33 indicates the axial direction of the loop fermentor, i.e. typically upwards.

Figure 15 shows a longitudinal section executed through the axis of a fifth embodiment of an inner loop fermentor according to the present invention. The rotation in the inner separation chamber 7 can be produced by spiral channels, e.g. as shown in Figure 5. Alternatively or in combination therewith screw channels can be provided in the interior of the separation chamber 7, possibly positioned higher than in figure 7 and figure 10. The upper part 71 of the fermentor gives rise to a more complete separation, so that less liquid leaves the fermentor through the outlet conduit 22 in connection with the centrifugal separation. In the Figure, 72 indicates an upper annular partition between the upper part 71 of the fermentor and the reaction volume 6, between which there is passage via the slit 73 which can be either annular or be constituted of tubes that may act as momentum exchange tubes if essentially downwards directed or obliquely tangential-downwards directed liquid nozzles are placed in or above them. Inlet means for air and liquid are not shown in the upper part or elsewhere, but such will at most applications be provided and can be of the same character as mentioned earlier in connection with the various Figures.

Figure 16 shows an embodiment which bears strong resemblance to the one shown in Figure 15, but with the difference, however, that there is no central, cylindrical tube in the interior of the separation volume 7 and that separated air and/or air-containing liquid as a consequence thereof is passed upwards out of the fermentor via the tube 22a. Apart from this deviation, what is mentioned above in connection with Figure 15 is otherwise valid also for the embodiment shown in Figure 16.

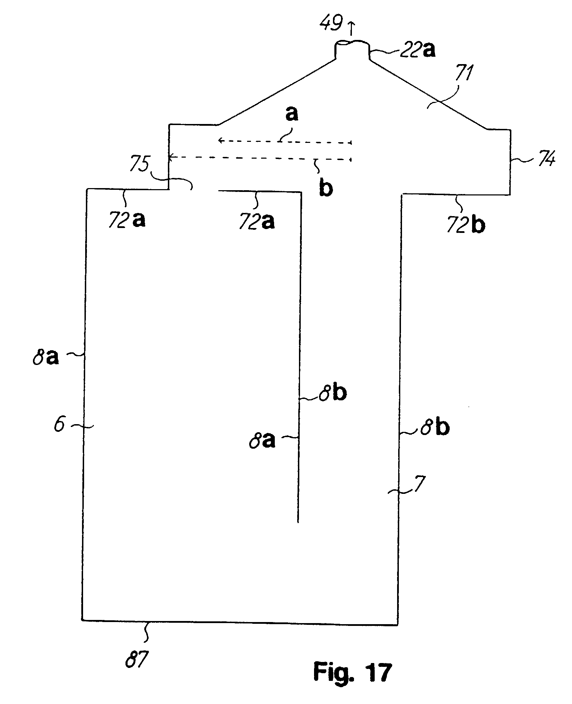

Figure 17 shows a longitudinal section executed through the axes of an outer loop fermentor according to the present invention. That the fermentor is an outer loop fermentor means that neither the ascending nor the descending part of the cycle is surrounded by the other part of the cycle. The fermentor shown has a working volume 6 which is defined in the vertical direction by a cylindrical container 8a-8a, below by a bottom wall 87, and above by a top plate 72a. The separation volume 7 is defined by another cylindrical container surface 8b-8b which at the one side adjoins the cylindrical container surface 8a, below by the bottom wall 87, and above by a cylindrical ring-plate 72b. The upper part 71 of the separation chamber 7 is essentially shaped as the upper part of the separation chamber 7 in Figure 16 with discharge of air and/or air-containing liquid 49 upwards out via the cylindrical tube 22a. The vertical side wall 74 of the upper part is, however, not rotationally symmetrical but has a spiral shape which increases from the minimal diameter a to the maximal diameter b. The upper space 71 is connected with the reaction chamber 6 via an opening 75 in the top plate 72a. At this place or above it, one or more nozzle devices (not shown) may be mounted for the introduction of a flow of liquid and air bubbles for hydropneumatic operation of the circulation in the fermentor. Apart from the distinctions mentioned above, the loop fermentor will in other respects function as the inner loop fermentors described above. An effective removal of introduced air bubbles will thus, for every passage of the cycle, take place in the cyclone separation chamber 71 or chambers 7 and 71.

Corresponding to the relationship between Figure 16 and Figure 17 any of the embodiments in Figures 1, 5, 7, 10, and 15 can be transformed into outer loops.

For further illustration and explanation of the invention the following shall be mentioned:

When the guiding plates 14, 16, 79, 80, 55, and 59 are so inclined in relation to the rotational axis of the cyclone separator of the loop that the average axial mass flow velocity of the liquid at the upper orifice edges 216, 48, and 62 of the inlet means is one third to one time the mathematical product of the average angular velocity of the liquid and the radius of the container wall 9 at the same position, an especially low level of turbulence is obtained in the cyclone separator as compared to the separatory centrifugal force thereof.

If a constriction of the cross sectional area is located in that part of the loop, where bubble-containing liquid is passed on towards the centrifugal separator, then a pressure above that of the atmosphere, caused either by overlaying liquid or possibly by operating the reactor throughout at elevated pressure, can totally or partly be transformed into an increase of the speed of the liquid, said increase making the centrifugal separator of the loop more effective. By this pressure reduction some of the pressure in the outlet gas is lost.

The constriction mentioned above can, if desired, be made more narrow, so that a larger pressure drop occurs. Hereby a pressure difference is achieved between the working volume and the centrifugal separator corresponding to a considerably increased height of the reactor (cf. ICI's well-known "pressure cycle" concept: high pressure during exploitation of gaseous substrate - low pressure during degassing of gaseous waste substances).

All ordinary, vertical loop reactors with upper debubbling have, of course, such a "pressure cycle" to a larger or lesser degree according to their height. Naturally, the pressure at the bottom is higher than the pressure at the top. The characteristic feature of the present invention is the possibility of using a constriction for the acceleration of liquid and pressure drop in the same and that the "pressure cycle" of tall loop reactors hereby can be created in lower reactors. This can be highly advantageous regarding construction costs, regional regulations, preservation considerations, etc.

Moreover, with the "pressure cycle" possibility of the present invention a uniformly high pressure can be established in the working volume of the reactor, so that an optimum hold-up can be maintained throughout therein, whilst the low pressure more suitable for the degassing is attained only at the degassing itself. As regards productivity and exploitation rate of substrate this is an advantage compared to the gradually decreasing pressure in ordinary loop reactors.

Correspondingly, a reverse pressure cycle with low reaction pressure and higher separation pressure can, if desired, be achieved by an enlargement of the cross sectional area before the separator.

Comminution into small bubbles of introduced gas can be effected in two ways, viz.: the gas can be passed through small openings into rapidly by-passing liquid, and bubbles already formed can be comminuted by strong velocity gradients in the liquid around the bubbles. The latter method may also be used on bubbles of gas which have not been introduced into the reactor or on bubbles which after introduction and comminution have coalesced.

If a centrifugal seperator utilizing high liquid speeds is employed, then introduced gas can be comminuted into bubbles by inlet orifices being placed in the outlet orifice of the separator for liquid being passed on through the reaction loop. If the high speed in the separator has been achieved by a constriction of the entrance or entrances to the separator, then the gas should preferably be introduced to the liquid leaving the separator before this liquid under deceleration re-attains the higher pressure in the working volume of the reaction loop.

Strong velocity gradients in the liquid around introduced gas can be created by introducing the gas in immediate connection with one or more nozzles in which liquid is having a pump pressure transformed into liquid velocity. If gas introducing means are mounted around such liquid nozzles, then it is also achieved that the gas is being introduced into rapidly flowing liquid.

By the use of liquid nozzles to obtain large velocity gradients in the liquid outside the nozzles, both the liquid pump pressure and the width of the nozzles are of importance. Thus, at a given available pump pressure it will be functionally advantageous to use nozzles of moderate width. Traditionally, the debouchments of nozzles for this purpose are either circular or short slit-shaped (example: Bayer AG's slit-shaped ejectors for aeration in water purification plants).

If it is desired to use small or narrow nozzles in a large reactor with high productivity, then nozzles having round or short-slit-shaped debouchments would have to be used in a large number, which implies a considerable construction cost. It is better to use nozzles having long-slit-shaped debouchments, which is naturally possible in a loop reactor operated at low bubble coalescence rate, in as much as the bubbles will then only have to be formed and delivered into the liquid which, due to the circulation of reactor contents in the reaction loop, is passing by the nozzles. This is in contrast to other reactors, in which larger liquid nozzles of more rounded shape are used for the production of liquid turbulence far from the nozzles so as to re-break coalesced bubbles, but at larger energy consumption.

It may be advantageous to have centrifugal fields both in the reaction volume and in the centrifugal separator of the loop. Ideally, the working volume is provided a radial dimension so large that the bubbles can obtain a fairly high and thus "k"-increasing radial velocity without this resulting in excessive separation of bubbles already in the working volume, whilst the centrifugal separator provides such a high proportion between the angular velocity of the reactor liquid and the radial thickness of this liquid that the separation here is rapid and effective. In addition, there should be large eddies in the working volume, as such eddies also counteract separation by interchanging (in radial direction) liquid volumes with many and few, respectively, bubbles, so that the bubbles are maintained fairly well distributed in the working volume, in spite of the centrifugal field.

If an inner reaction loop fermentor having a centrifugal separator in the inner part of the loop is used, then the reactor liquid will still be in rotation at its entry into the outer part of the loop, unless this has been prevented by the design of the fermentor. This natural, through conservation of momentum created liquid rotation in the outer part of the loop can, if desired, be reduced by making the rotation of the liquid acircular, so that turbulences are created, and it can be either increased or reduced by supplying tangentially directed momentum, in addition to the axial power supply to the reactor liquid (for causing the liquid circulation in the loop), by changed inclination of the momentum supplying means or through alternative momentum supply in tangential direction.

For an outer reaction loop fermentor the above can also be said almost word-for-word, when the working volume of the loop essentially consists of an axial continuation of the centrifugal separator. If, on the contrary, the centrifugal separator essentially is an axial continuation of the working volume, then the momentum transfer into rotation of the liquid in the working volume will be determined by the cross sectional area of the return conduit and how far from the center-axis of the working volume the return takes place.

A particular case is when the reactor is being supplied one or more relatively expensive gaseous substances like e.g. $CH_4$, $O_2$, and $NH_3$ which should be exploited nearly 100% in a good reactor and also a less expensive substance like e.g. atmospheric air which due to its mixed composition cannot be expected to be fully exploited. In such a situation it will be profitable first to introduce the expensive gases as very small bubbles (e.g. natural gas or biogas) followed further on in the loop by the less expensive gas in the form of larger bubbles. In this way the expensive gases can be exploited to a higher degree, while the less expensive gas during its removal will only entrain remnants of the expensive gases to a minimum degree.

The situation mentioned above may be relevant at e.g. the cultivation of a methanotrophic bacteria culture using methane-containing gas (e.g. natural gas or biogas) as the only or the major carbon source and possibly also $NH_3$ as nitrogen source or supplement and/or purified $O_2$ (e.g. of industrial quality) as $O_2$ source or $O_2$ supplement.

When cultivating such a culture there is the complication that at failing control, or if the consumption by the culture fails for one or another reason, then inflammable, methane- and oxygen-containing gas may be debubbled from the reactor liquid. This implies the risk of an increase of pressure created by a combustion in the reactor, whereby the reactor may be damaged and perhaps even eject explosion fragments causing danger in the surroundings. It is especially disadvantageous to have a large, upper chamber for outlet gases released in the reactor, because this chamber will necessarily have to be equipped with relief means for explosion pressures, e.g. blast caps, whereby cultivation at elevated pressure will be complicated considerably.

These problems can be redressed by the present invention, since the centrifugal separator can be placed in the middle of the reactor or even at the bottom thereof, at the same time as debubbled gas is passed off in downwards direction. Hereby all exhaust gas will be surrounded sidewards and possibly also upwards by the liquid-filled working volume of the reactor, at the same time as the total amount of released gas inside the fermentor is reduced to a very small fraction of what would otherwise be the case.

The above facts make it possible to construct the reactor in such a manner that no explosion pressure relief means are actuated in a liquid-filled and working reactor in case of burning of gas released from the reactor.

The explosion pressure relief means may be placed in subterranean means for conducting the exhaust gases away, and due to this subterranean position relief can be effected into a subterranean chamber placed there for this purpose. Additionally the chamber can be kept ventilated with sterile-filtered air at a suitable pressure. Thereby it becomes possible and advantageous to use e.g. one or more foil valves for the relief, so that the connection between the means for conducting exhaust gases away and the relief chamber closes again after a relief. This avoids that the relief chamber is filled with inflammable gas and also the risk of introducing an infection into the culture in connection with an occurred relief, since the relief is into a sterile chamber.

A possible burning of released gas can thus be turned into a hazard-less event by the reactor of the present invention without any need for repairs regarding e.g. blast caps and without any risk of infection into the culture, if only the relief chamber is maintained at a pressure which will keep the connection to the means for conducting exhaust gas away sufficiently tightly closed before and after relief, so that the ventilation of the relief chamber is capable to prevent accumulation of inflammable gas in the relief chamber. It is especially advantageous to avoid having to climb a traditional reactor to mount new blast caps, just after a burning has taken place, because the reactor cannot be operated neither automatically nor manually, when the composition of the exhaust gases cannot be measured due to the now open blast caps. Alternatively, it is advantageous not to need to cut off the gas for the reactor, risking that the complete content of bacteria of the reactor die of the cause which just moments before caused their gas consumption to be so reduced that a burning could take place, just because the operator(s) is (are) afraid of climbing the reactor to mount new blast caps, while inflammable gas is still being debubbled from the culture.

In addition to the above serious problems being completely solvable by the present invention, it will be possible by constructing the relief chamber and its valves as mentioned to cultivate without any problems at elevated pressure in the reactor, if only the pressure in the relief chamber is kept somewhat higher. For example hinge-mounted flap valves can be used in stead of foil valves, if that is considered appropriate. Also, valves can be provided with actuators (it may e.g. be solenoid valves), which are actuating opening and closure of the valves at preset pressure limits.

That cultivation can be carried out at elevated pressure means that the increased productivity attached therewith can be achieved also in reactors, in which there is a risk of occurrence of inflammable exhaust gases.

In addition to the embodiments of the invention as defined in the appended claims the following embodiments are also preferred:

I. A process wherein one or more relatively expensive gaseous substances and also a less expensive gas is being introduced into the reaction volume of the loop, the less expensive gas, preferably atmospheric air, introduced ahead the other gaseous substance(s).

II. A process wherein the expensive gaseous substance(s) include(s) methane-containing gas and/or ammonia and/or purified oxygen.

III. A loop reactor wherein the centrifugal separator (7) is a cyclone separator having one or more inlet debouchment(s) (12, 112, 46) connected with inlet means (9, 22, 59 (Figs. 7, 8, and 9)) providing helical or screw-shaped channels (58) having twisted guiding plates (59 (Figs. 8 and 9)) or curved guiding plates (59 (Figs. 12, 13, and 14)), said guiding plates optionally being positioned within the separation volume (7), preferably in the lower part thereof.

IV. A loop reactor wherein the centrifugal separator (7) is a cyclone separator having one or more inlet debouchment(s) (12, 112, 46) connected with inlet means (43, 55, 87 (Figs. 5 and 6)) providing spiral-shaped channels (44) having side walls (55) extending out into the reaction volume (6) of the loop.

V. A loop reactor wherein the centrifugal separator (7) is a cyclone separator having one or more inlet debouchment(s) (12, 112, 46) connected with inlet means (14, 114, 16, 116, 79, 179, 80, 180, 87 (Figs. 1 and 2)) providing spirally and helically shaped channels having guiding plates (14, 114, 16, 116, 79, 179 (Figs. 1 and 2)) extending out into the reaction volume (6) of the loop.

VI. A loop reactor wherein the guiding plates (14, 114, 16, 116, 79, 179, 80, 180, 55, and 59) in the cyclone separator are so inclined in relation to the rotational axis of the cyclone separator (7) of the loop that the average axial mass flow velocity of the liquid at the upper edges (214, 216, 48, 62) of the inlet means is 1/3 to. 1 time the mathematical product of the average angular velocity of the liquid and the radius of the container wall (9) at the same position.

VII. A loop reactor wherein the circulation flow is directed upwards in the cyclone separator (7) and downwards in the reaction volume (6), and that an extra set of nozzle devices (65a, 65b) are provided as a rim exteriorly to the ring-passage (2) for downwards, optionally vertically downwards, introduction of liquid into the reaction volume (6), said nozzl.e devices (65a, 65b) optionally also supplying bubbles of gas, preferably air, different from the gas optionally delivered from the other nozzle devices (30), and said nozzle devices (65a, 65b) optionally being so designed as to supply sufficient extra energy-power, additional to the energy-power supplied by the other nozzle devices (30), for the operation of both the circulation in the reactor cycle and the separation in the cyclone separator.

VIII. A loop reactor wherein a blocking means (69) is provided in the upper part of the reaction volume (6) and momentum exchange tubes (66a, 66b), optionally provided with diffusers (67), are placed in said blocking means (69), in line with the nozzle devices (65a, 65b) mounted above.

IX. A loop reactor which is an outer loop reactor, preferably having one or more nozzle devices placed at the entrance to the reaction volume (6) for introducing liquid and optionally one or more gaseous substances which is (are) torn out into gas bubbles of desired size, said nozzle devices optionally being so designed as to supply sufficient energy-power for operation of both the circulation in the reactor cycle and the separation in the cyclone separator.

X. A loop reactor which has an upper part (71) providing a separation chamber above and in continuation of the separation volume (7), said chamber being wider than the separation volume (7).

**Claims**

1. A process for effecting a chemical, biochemical, or biological reaction or production in a loop reactor, in or into which immiscible phases are produced or introduced, respectively, during the circulation of the contents of the reactor, **characterized** in that for each cycle passage through the loop all or essentially all the reactor contents are passed through a centrifugal separator for generation of at least two fractions having different contents of at least two immiscible phases, one of which fractions continues through the loop, while the other is drained off the loop.

2. A process according to claim 1, **characterized** in that said reactor contents are passed into said centrifugal separator at its bottom, and that said at least two fractions having different contents of at least two immiscible phases are generated by passing the reactor contents through the centrifugal separator in an upwards directed helical rotation and are separated physically from each other by the lower edge of a cylindrical separatory wall at the upper end of the centrifugal separator and both passed out thereof.

3. A process according to claim 2, **characterized** in that the centrifugal separator is a cyclone separator and that the reactor contents passed into said cyclone separator at its bottom optionally already having been imparted an axial and/or rotational mass flow velocity component at their introduction into the separation chamber of said cyclone separator.

4. A process according to any of claims 1 to 3, **characterized** in that said at least two immiscible phases are liquid and gas, respectively.

5. A process according to claim 4, **characterized** in that the process is an aerobic fermentation, in which bubbles of oxygen-containing gas and possibly other gaseous substances are continously introduced at the entrance of a reaction volume in the loop, and that a gas fraction and/or a gas bubble-containing liquid fraction is (are) continually withdrawn at the exit of said centrifugal separator and conducted away from the loop.

6. A process according to claim 5, **characterized** in that said oxygen-containing gas is atmospheric air, and that the withdrawn fraction consists of oxygen-depleted, carbon dioxide-containing gas and optionally other gaseous substances and/or a liquid fraction containing bubbles thereof which are sent to an outer centrifugal separator in which gas and liquid phases are effectively separated, after which the liquid phase in its entirety or in part is recirculated to the reactor cycle, possibly added supplementary liquid with contents of nutrients, salts and micronutrients, where it together with air and possibly other gaseous substances is introduced into the fermentation liquid as a momentum flow that tears out the introduced air and possibly other gaseous substances into bubbles of the size desired.

7. A loop reactor for performing the process according to claim 1 and comprising at least one loop with a reaction volume (6), propulsion means (30, 65a, 65b) for operating the circulation in the loop, and at least one centrifugal separator (7), **characterized** in that the said centrifugal separator (7) is an integrated part of said loop, thus receiving all or essentially all the reactor contents for each cycle passage of the reactor contents through the loop, whereby said contents are divided into at least two fractions having different contents of at least two different costituents, and that said centrifugal separator has means (2) for passing one of said fractions further on in the loop, and means (22, 22a) for draining off the other of said fractions.

8. A loop reactor according to claim 7, **characterized** in that said centrifugal separator (7) is a cyclone separator having one or more debouchments (12, 112, 46) at its bottom for passing said reactor contents into it and at its top both a cylindrical ring-passage (2) for the fraction continuing on through the loop and either an annular opening (3) or another outlet (22a) for the other fraction being drained off the loop.

9. A loop reactor according to claims 7 or 8, **characterized** in that said cyclone separator has an axially positioned cylindrical member (22) which extends through all or the main part of the cyclone chamber (7), optionally being hollow and optionally having an opening at its top for withdrawing a flow of the inner of the separated fractions which in that case is passed through the bottom (87) of the separation chamber (7).

10. A loop reactor according to claim 8, **characterized** in that the means for separation of the two fractions generated in the cyclone separator is a cylindrical separatory wall (21, 22a) protruding from the top (89) of the separator volume (7) or the top wall (88) of the reactor, and that the upper part of the wall (9) of the separator volume together with either said separatory wall (21, 22a) or the top wall (88) of the reactor define a ring-passage (2) for the outer fraction generated in the separator and continuing further on in the reactor loop.

11. A loop reactor according to any of claims 8 to 10, **characterized** in that the reactor is an inner loop reactor, in which said cyclone separator is an integral part of the inner part (7) of the loop, while the outer part (6) of the loop essentially constitutes the reaction volume.

12. A loop reactor according to claim 11, **characterized** in that the means (2) for passing one of said fractions further on in the loop is a ring-passage and that nozzle devices (30) are positioned as a rim in this ring-passage, preferably directed obliquely upwards and preferably spanning all across the ring-passage, for introducing liquid and optionally one or more gaseous substances which is (are) torn out into gas bubbles of desired size, said nozzle devices optionally being so designed as to supply sufficient propulsion force for operation of both the circulation in the reactor cycle and the separation in the cyclone separator.

13. A loop reactor according to claims 10 or 12, **characterized** in that said ring-passage (2) continues into an annular passage (90) which is provided with a diffuser (68).

14. A loop reactor according to any of claims 7 to 13, **characterized** in that the means (22, 22a) for draining off the other of said fractions are connected with a conduit (49) for passing this fraction to a further external separator (23), preferably a centrifugal separator, for complete separation of the said two immiscible phases of this fraction, and that one of the separated phases or a part of it is optionally recirculated to the loop reactor by means of a conduit (25), pump(s) (28), and a further conduit (conduits) (50).

**Patentansprüche**

1. Verfahren zur Ausführung einer chemischen, biochemischen oder biologischen Reaktion oder Herstellung in einem Schleifenreaktor, in dem bzw. in den während der Zirkulation des Reaktorinhalts unmischbare Phasen hergestellt bzw. eingeleitet werden, dadurch gekennzeichnet, daß für jeden Kreislauf durch die Schleife der gesamte oder im wesentlichen der gesamte Reaktorinhalt einen Zentrifugalabscheider zur Erzeugung mindestens zweier Fraktionen mit unterschiedlichen Anteilen von mindestens zwei unmischbaren Phasen durchläuft, wobei eine der Fraktionen die Schleife weiter durchläuft, während die andere aus der Schleife abgelassen wird.

2. Verfahren nach Anspruch 1, dadurch gekennzeichnet, daß der Reaktorinhalt in den Zentrifugalabscheider an dessen Boden eingetragen wird und daß mindestens zwei Fraktionen mit unterschiedlichen Anteilen von mindestens zwei unmischbaren Phasen erzeugt werden, indem man den Reaktorinhalt in aufwärts gerichteter schraubenförmiger Drehung den Zentrifugalabscheider durchlaufen läßt, und durch die Unterkante einer zylinderförmigen Scheidewand am oberen Ende des zentrifugalabscheiders physikalisch voneinander getrennt und ausgetragen werden.

3. Verfahren nach Anspruch 2, dadurch gekennzeichnet, daß der zentrifugalabscheider ein Zyklonabscheider ist und daß dem Reaktorinhalt, der in den Zyklonabscheider an dessen Boden eingetragen wird, wahlweise bereits bei seinem Einleiten in die Trennkammer des Zyklonabscheiders eine Axial- und/oder Rotationskomponente der Massenströmungsgeschwindigkeit erteilt worden ist.

4. Verfahren nach einem der Ansprüche 1 bis 3, dadurch gekennzeichnet, daß die mindestens zwei unmischbaren Phasen eine Flüssigkeit bzw. ein Gas sind.

5. Verfahren nach Anspruch 4, dadurch gekennzeichnet, daß das Verfahren eine aerobe Fermentierung ist, bei der Blasen aus sauerstoffhaltigem Gas und möglicherweise anderen gasförmigen Substanzen am Eingang zu einem Reaktionsvolumen ständig in die Schleife eingeleitet werden, und daß eine Gasfraktion und/oder eine gasblasenhaltige Flüssigkeitsfraktion ständig am Ausgang des Zentrifugalabscheiders abgesaugt und von der Schleife weggeführt werden.

6. Verfahren nach Anspruch 5, dadurch gekennzeichnet, daß das sauerstoffhaltige Gas athmosphärische Luft ist und daß die abgesaugte Fraktion aus sauerstoffarmem, kohlendioxidhaltigem Gas und wahlweise aus anderen gasförmigen Substanzen und/oder einer flüssigen Fraktion besteht, die Blasen dieser Substanzen enthält, die einem äußeren zentrifugalabscheider zugeführt werden, in dem die Gasphase und die flüssige Phase wirksam getrennt werden, wonach die flüssige Phase ganz oder teilweise in den Reaktorkreislauf zurückgeführt wird, möglicherweise unter Zugabe von Ergänzungsflüssigkeit mit Anteilen an Nährstoffen, Salzen und Mikronährstoffen, wo sie zusammen mit Luft und möglicherweise anderen gasförmigen Substanzen in die Fermentierungsflüssigkeit als Impulsströmung eingeleitet wird, welche die eingeleitete Luft und möglicherweise andere gasförmige Substanzen in Blasen von der gewünschten Größe mitreißt.

7. Schleifenreaktor zur Ausführung des Verfahrens nach Anspruch 1, der mindestens eine Schleife mit einem Reaktionsvolumen (6), eine Antriebseinrichtung (30, 65a, 65b) zur Betreiben der Zirkulation in der Schleife und mindestens einen Zentrifugalabscheider (7) aufweist, dadurch gekennzeichnet, daß Zentrifugalabscheider (7) ein integrierter Teil der Schleife ist, so daß er bei jedem Kreislauf des Reaktorinhalts durch die Schleife den gesamten oder im wesentlichen den gesamten Reaktorinhalt aufnimmt, wodurch der Inhalt in mindestens zwei Fraktionen mit unterschiedlichen Anteilen von mindestens zwei verschiedenen Bestandteilen getrennt wird, und daß der Zentrifugalabscheider eine Einrichtung (2) zum Weiterleiten einer der Fraktionen in der Schleife und eine Einrichtung (22, 22a) zum Ablassen der anderen Fraktion aufweist.

8. Schleifenreaktor nach Anspruch 7, dadurch gekennzeichnet, daß der Zentrifugalabscheider (7) ein Zyklonabscheider ist, der an seinem Boden eine oder mehrere Einmündungen (12, 112, 46) zum Einlassen des Reaktorinhalts und an seinem oberen Ende sowohl einen zylinderförmigen Ringkanal (2) für die weiter durch die Schleife laufende Fraktion als auch entweder eine ringförmige Öffnung (3) oder einen anderen Auslaß (22a) für die andere Fraktion aufweist, die aus der Schleife abgelassen wird.

9. Schleifenreaktor nach einem der Ansprüche 7 oder 8, dadurch gekennzeichnet, daß der Zyklonabscheider ein axial angeordnetes zylinderförmiges Element (22) aufweist, das sich durch die gesamte oder durch einen Teil der Zyklonkammer (7) erstreckt, wahlweise hohl ist und wahlweise an seinem oberen Ende eine Öffnung zum Absaugen einer Strömung der inneren von den getrennten Fraktionen aufweist, die in diesem Falle durch den Boden (87) der Trennkammer (7) geleitet wird.

10. Schleifenreaktor nach Anspruch 8, dadurch gekennzeichnet, daß die Einrichtung zum Trennen der beiden im Zyklonabscheider erzeugten Fraktionen eine zylinderförmige Scheidewand (21, 22a) ist, die vom Dach (89) des Abscheidervolumens (7) oder der oberen Wand (88) des Reaktors vorsteht, und daß der obere Teil der Wand (9) des Abscheidervolumens zusammen entweder mit der Scheidewand (21, 22a) oder mit der oberen Wand (88) des Reaktors einen Ringkanal (2) für die äußere Fraktion bildet, die im Abscheider erzeugt wird und die Reaktorschleife weiter durchläuft.

11. Schleifenreaktor nach einem der Ansprüche 8 bis 10, dadurch gekennzeichnet, daß der Reaktor ein Innenschleifenreaktor ist, in dem der Zyklonabscheider ein integrierter Teil des inneren Teils (7) der Schleife ist, während der äußere Teil (6) der Schleife im wesentlichen das Reaktionsvolumen bildet.

12. Schleifenreaktor nach Anspruch 11, dadurch gekennzeichnet, daß die Einrichtung (2) zum Weiterleiten einer der Fraktionen in der Schleife ein Ringkanal ist und daß in diesem Ringkanal Düsenvorrichtungen (30) kranzförmig angeordnet sind, die vorzugsweise schräg nach oben gerichtet sind und vorzugsweise den gesamten Ringkanal überspannen und zum Einleiten von Flüssigkeit und wahlweise einer oder mehrerer gasförmiger Substanzen dienen, die in Gasblasen der gewUnschten Größe mitge rissen werden, wobei die Düsenvorrichtungen wahlweise so konstruiert sind, daß sie eine ausreichende Antriebskraft für den Betrieb sowohl der Zirkulation im Reaktorkreislauf als auch der Trennung im Zyklonabscheider liefern.

13. Schleifenreaktor nach einem der Ansprüche 10 oder 12, dadurch gekennzeichnet, daß der Ringkanal (2) sich in einen ringförmigen Kanal (90) fortsetzt, der mit einem Diffusor (68) ausgestattet ist.

14. Schleifenreaktor nach einem der Ansprüche 7 bis 13, dadurch gekennzeichnet, daß die Einrichtung (22, 22a) zum Ablassen der anderen Fraktion mit einer Röhre (49) verbunden ist, um diese Fraktion zu einem weiteren äußeren Abscheider (23), vorzugsweise einem Zentrifugalabscheider, zur vollständigen Trennung der beiden unmischbaren Phasen dieser Fraktion weiterzuleiten, und daß eine der getrennten Phasen oder ein Teil davon wahlweise mit Hilfe einer Röhre (25), einer oder mehrerer Pumpen (28) und einer oder mehrerer weiterer Röhren (50) zum Schleifenreaktor zurückgeführt wird.

**Revendications**

1. Un procédé pour effectuer une réaction ou une production chimique, biochimique, ou biologique, dans un réacteur à boucle, dans lequel sont produites où introduites des phases non miscibles, pendant la circulation du contenu du réacteur, caractérisé en ce que, pour chaque passage de cycle, on fait passer la totalité, ou sensiblement la totalité, du contenu du réacteur, par un séparateur centrifuge, pour générer au moins deux fractions présentant des contenus différents d'au moins deux phases non miscibles, l'une de ces fractions continuant à travers la boucle, tandis que l'autre fraction est évacuée de la boucle.

2. Un procédé selon la revendication 1, caracterisé en ce que l'on fait passer ledit contenu du réacteur dans ledit séparateur centrifuge, par le fond de ce dernier, et en ce que lesdites au moins deux fractions présentant des contenus différents d'au moins deux phases non miscibles, sont générées en faisant passer le contenu du réacteur à travers le séparateur centrifuge par une rotation hélicoïdale dirigée vers le haut, et on les sépare physiquement l'une de l'autre par le bord inférieur d'une paroi cylindrique de séparation à l'extrémité supérieure du séparateur centrifuge et on fait sortir les deux fractions.

3. Un procédé selon la revendication 2, caractérisé en ce que le séparateur centrifuge est un séparateur à cyclone et en ce que le contenu du réacteur qui passe dans ledit séparateur à cyclone par son fond reçoit déjà, le cas échéant, une impulsion provoquant une composante axiale et/ou rotationnelle de vitesse d'écoulement de masse à son introduction dans la chambre de séparation dudit séparateur à cyclone.

4. Un procédé selon l'une quelconque des revendications 1 à 3, caractérisé en ce que lesdites au moins deux phases non miscibles sont respectivement du liquide et du gaz.

5. Un procédé selon la revendication 4, caractérisé en ce que le procédé est une fermentation aérobic, dans laquelle des bulles de gaz contenant de l'oxygène, et le cas échéant d'autres substances gazeuses, sont introduites en continu à l'entrée d'un volume de réaction dans la boucle, et en ce qu'une fraction gazeuse et/ou une fraction liquide contenant des bulles de gaz est (sont) extraite(s) en continu à la sortie dudit séparateur centrifuge et est (sont) évacuée(s) de la boucle.

6. Un procédé selon la revendication 5, caractérisé en ce que ledit gaz contenant de l'oxygène est de l'air atmosphérique, et en ce que la fraction extraite consiste en un gaz appauvri en oxygène et contenant du dioxyde de carbone, et le cas échéant d'autres substances gazeuses, et/ou une fraction liquide contenant des bulles de gaz qui sont envoyés à un séparateur centrifuge extérieur dans lequel les phases gazeuses et liquides sont effectivement séparées, après quoi la phase liquide, en totalité ou en partie, est remise en circulation vers le cycle des réacteurs, le cas échéant du liquide complémentaire est ajouté ayant un contenu d'aliment, de sel et d'oligo-éléments, et où il est introduit avec de l'air et le cas échéant d'autres substances gazeuses dans le liquide de fermentation, sous la forme d'un courant actif qui extrait l'air introduit, et le cas échéant d'autres substances gazeuses sous la forme de bulles présentant la taille souhaitée.

7. Un réacteur à boucle pour mettre en oeuvre le procédé selon la revendication 1, et comprenant au moins une boucle avec un volume de réaction (6), des moyens de propulsion (30, 65a, 65b) pour provoquer la circulation dans la boucle, et au moins un séparateur centrifuge (7), caractérisé en ce que ledit séparateur centrifuge (7) fait partie intégrante de ladite boucle, recevant ainsi la totalité ou sensiblement la totalité du contenu du réacteur pour chaque passage de cycle du contenu du réacteur à travers la boucle, de telle manière que ledit contenu soit divisé en au moins deux fractions présentant des contenus différents d'au moins deux constituants différents, et en ce que ledit séparateur centrifuge comporte des moyens (2) pour faire passer l'une desdites fractions plus loin dans la boucle, et des moyens (22, 22a) pour évacuer à l'extérieur l'autre desdites fractions.

8. Un réacteur à boucle selon la revendication 7, caractérisé en ce que ledit séparateur centrifuge (7) est un séparateur à cyclone présentant une ou plusieurs bouches d'évacuation (12, 112, 46) de son fond, pour faire passer ledit contenu du réacteur dans le réacteur et à sa partie supérieure, à la fois un passage annulaire cylindrique (2) pour la fraction continuant à travers la boucle, et soit une ouverture annulaire (3) ou une autre sortie (22a) pour l'autre fraction évacuée hors de la boucle.

9. Un réacteur à boucle selon les revendications 7 ou 8, caractérisé en ce que ledit séparateur à cyclone comporte un organe cylindrique positionné axialement (22) qui s'étend sur la totalité ou la majeure partie de la chambre de cyclone (7) et qui, le cas échéant, est creux et, le cas échéant, présente une ouverture à sa partie supérieure, pour extraire un courant de la plus à l'intérieur des fractions séparées, que l'on fait passer dans ce cas à travers le fond (87) de la chambre de séparation (7).

10. Un réacteur à boucle selon la revendication 8, caractérisé en ce que les moyens pour séparer les deux fractions générées dans le séparateur à cyclone sont constitués par une paroi cylindrique de séparation (21, 22a) faisant saillie de la partie supérieure (89) du volume de séparation (7); ou bien de la paroi supérieure (88) du réacteur, et en ce que la partie supérieure de la paroi (9) du volume de séparation en conjugaison avec soit ladite paroi de séparation (21, 22a) soit la paroi supérieure (88) du réacteur définit un passage annulaire (2) pour la fraction la plus à l'extérieur générée dans le séparateur et continuant plus loin dans la boucle de réacteur.

11. Un réacteur à boucle selon l'une quelconque des revendications 8 à 10, caractérisé en ce que le réacteur est un réacteur à boucle intérieure, dans lequel ledit séparateur à cyclone est partie intégrante de la partie la plus intérieure·(7) de la boucle, tandis que la partie extérieure (6) de la boucle constitue sensiblement le volume de réaction.

12. Un réacteur à boucle selon la revendication 11, caractérisé en ce que les moyens (2) pour faire passer l'une desdites fractions plus loin dans la boucle est un passage annulaire, et en ce que des dispositifs de buses (30) sont positionnés pour constituer un bord dans ce passage annulaire, sont préférablement dirigés obliquement vers le haut et s'étendent préférablement à travers la totalité du passage annulaire, pour introduire du liquide et, le cas échéant, une ou plusieurs substances gazeuses qui est (sont) extraite(s) sous la forme de bulles de gaz de la taille souhaitée, lesdits dispositifs de buses étant le cas échéant, réalisés de façon à assurer une force de propulsion suffisante pour réaliser à la fois la circulation dans le cycle de réacteur et la séparation dans le séparateur à cyclone.

13. Un réacteur à boucle selon les revendications 10 ou 12, caractérisé en ce que ledit passage annulaire (2) se prolonge en un passage annulaire (90) qui est muni d'un diffuseur (68).

14. Un réacteur à boucle selon l'une quelconque des revendications 7 à 13, caractérisé en ce que les moyens (22, 22a) pour évacuer l'autre desdites fractions sont reliés à un conduit (49) pour faire passer ces fractions vers un autre séparateur extérieur (23), de préférence un séparateur centrifuge, pour la séparation complète desdites deux phases non miscibles de cette fraction, et en ce que l'une des phases séparées ou une partie de celle-ci est, le cas échéant, recirculée vers le réacteur à boucle au moyen d'un conduit (25), de pompes (28) et d'un autre conduit ou (d'autres conduits) (50).

Fig. 1

**Fig. 2**

**Fig. 3**

**Fig. 4**

Fig. 5

Fig. 6

Fig. 7

Fig. 8

Fig. 9

EP 0 510 010 B1

**Fig. 10**

**Fig. 11**

**Fig. 12**

**Fig. 13**

**Fig. 14**

EP 0 510 010 B1

Fig. 15

Fig. 16

25

**Fig. 17**